# EUROPEAN PATENT APPLICATION

(11) **EP 0 586 353 A1**
(43) Date of publication of application: **09.03.1994**
(21) Application number: 93850160.8
(22) Date of filing: 20.08.1993
(51) Int. Cl.: H01S 3/02, A61B 17/36, A61N 5/06

(54) **Arrangement for emission of light or laser light**

(30) Priority: 31.08.1992 SE 9202494
(71) Applicant: Gustavsson, Morgan, S-413 01 Göteborg (SE)
(72) Inventor: Gustavsson, Morgan, S-413 01 Göteborg (SE)
(74) Representative: Kristiansen, Alf P.

(57) **Abstract**

The invention relates to an arrangement for emission of light or laser light. It is characterized in that it comprises a handheld cover which houses an exchangeable, preferably rod-shaped, fluorescent cell or wave guide laser. The arrangement further comprises supply means for energy to the cell (1) or the wave guide conductor, and output means for emitted light or laser light.

## Description

### TECHNICAL FIELD:

The present invention relates to a handheld device for emission of light or laser light which is intended for light or laser light treatment primarily of skin affections on humans.

### BACKGROUND OF THE INVENTION:

Arrangements for light or laser light treatment of skin affections are previously known. These consist principally of a statically mounted or movable central box containing a power aggregate and a cooling arrangement. The power aggregate in the box transforms electrical energy into light beams of desired frequency which are conducted via a light conductor to a handheld light emission apparatus with which the light beams can be directed towards a desired location. To be able to maintain the temperature in the box and other parts of the arrangement at a suitable level, some kind of water cooling is normally provided. In the box the desired light is normally created by having an electric lamp irradiate a fluorescent substance which is excited and emits light of the desired wavelength. The fluorescent substance is usually present in a solution and is agitated by means of a pump or the light. An example of such a device is described in US Patent No. 5 066 293. The light from these arrangements is conducted via so-called light conductors to the handheld light emitter.

Light conductors from such light-creating devices as described above display large energy losses, especially at the so-called light coupling where the light from the fluorescent cell is fed into the light conductor. The reason for this is primarily the wide light cone which is dissipated from such fluorescent cells or wave guide lasers, or friction losses when using fibre bunches. The light beam which emerges from the handheld light emitter is typically a converging parallel beam and the light conductor should therefore not come into direct contact with the area of the skin that is to be treated with these rays. A distance of 1-2 cm from the skin is usual.

### SUMMARY OF THE INVENTION:

There has therefore been a long-felt need to be able to generate the desired light close to the skin area so that large energy losses can be avoided.

In accordance with the present invention, this is achieved by an arrangement for emission of light or laser light which is characterized in that it comprises a longitudinal cover adapted to be handheld, which cover houses an exchangeable, preferably rod-shaped, fluorescent cell with or without an optical resonator and supply means for energy to the cell and output means for emitted light or laser light.

Preferably, the cell in the arrangement consists of one or more dyes in plastics, or dissolved in a solvent or the like in a glass pipe, plastic pipe or the like, or as a self-sustaining element.

The plastic material may consist of polymethylmetacrylate or hydroxypropylacrylate and the solvent may consist of polyethyleneglycol, ethyleneglycol, ethanol, methanol etc., whilst the dye can be one which is emitting in the ultraviolet, the visible or infra-red spectrum, for example cumarines or rhodamines.

The cell according to the invention can be mounted in the arrangement by means of a rubber ring or the like which is pushed on the glass pipe.

The glass pipe or the cell can preferably be provided with a mirror in at least one end, whereas at the other end it can be provided with an annular mirror. It can according to the invention be advantageous that the rear end is provided with a spectrally partial mirror while the cell is fed with light energy axially from the rear end.

Preferably, the glass pipe at the forward end can be without a mirror and a glass plug in the pipe, which entraps the fluorescent material, is provided with a mirror.

The cell having the fluorescent material can further suitably be fed with light energy laterally from a lamp.

According to a preferred embodiment of the invention, the supply means for the energy suitably includes a device for supply of electrical energy having a high voltage, electrical energy having a low voltage or light.

### BRIEF DESCRIPTION OF THE DRAWINGS:

The invention will be described in the following in greater detail with reference to the attached drawings in which:
- Fig. 1: is an axial sectional view through a handheld device according to the invention, and
- Fig. 2: shows in a longitudinal section the cell itself with the fluorescent material.

### BEST MODE OF CARRYING OUT THE INVENTION:

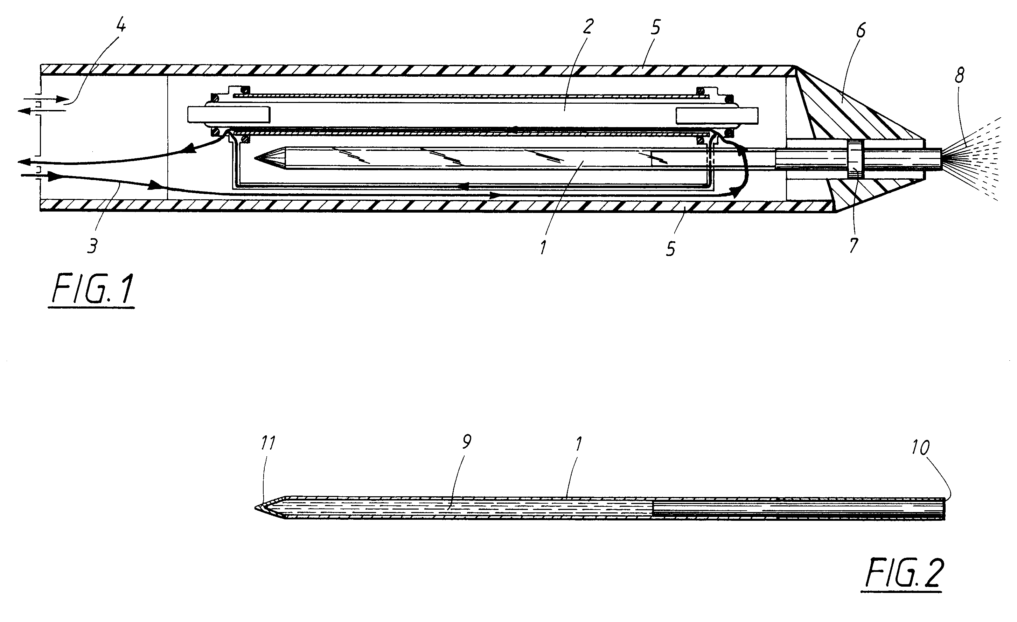

Fig. 1 shows an arrangement according to the invention having an exchangeable fluorescent rod-shaped cell 1 and a lamp 2 which illuminates the cell 1 laterally. The lamp 2 is of a conventional kind and is not described further here. It is provided with a reflector at the side so that the light is forced to radiate towards the fluorescent cell 1. The lamp is located in a glass pipe which allows for circulation of cooling water. The cooling water is also extended to the area around the fluorescent cell 1 and follows in principle the line 3 which is indicated with arrows. The intake for energy is schematically denoted by reference numeral 4 and is located at one end, the so called rear end, of the handheld apparatus. The cell 1 is surrounded by air so that the refractive index at the transition from air to glass and vice versa will be decisive for the function of the arrangement. The arrangement is also surrounded by a cover 5 which, due to the water cooling, should have a temperature of 10-15°C on its inside. At the forward end of the device there is an end piece 6 which projects somewhat into the cover 5. An extension of the fluorescent cell 1 projects through the end piece 6. The cell is also held in the end piece by means of the cell being provided with a rubber ring 7 which fits in a recess in the plug 6. In this manner, the cell 1 can easily be exchanged. It is necessary merely to pull the cell 1 out from the end piece 6 and to replace it with another cell. As further appears from the drawing, a light beam 8 radiates from the fluorescent cell when electric current is applied to the lamp 2. This light, which can be coherent or non coherent, is shown in the shape of a cone with a dispersion of about 45°. In the present case the cell 1 is only suspended at one point by means of the rubber ring 7, but it is suitably inserted at the other end in some kind of support (which is not shown in the drawing) to anchor it firmly.

Fig. 2 shows in more detail the fluorescent cell 1. This consists of a hollow glass rod 1 in which a fluorescent material 9 in the form of, for example, some kind of plastics which is mixed with a colour or possibly a solvent with a dissolved dye is present. The plastic, which is moulded and in a solid shape, can easily be inserted. It can suitably consist of polymethylacrylate or hydroxypropylacrylate. Other transparent plastics are also possible. As examples of solvents, ethanol or methanol can be mentioned, and as examples of dyes cumarines for removing of melamine pigments and rhodamines for removing of superficial vessels in a skin area can be mentioned. Other dyes and solvents are of course also possible. Fluorescence in the whole visible, infra red and ultra violet spectrum and the so called NIR-area spectrum is also conceivable (NIR = Near Infra Red). If the fluorescent material 9 is bound to a solid body, this may be provided with a mirror at the surface which faces forwardly, that is to the right in Fig. 2. The light beams from the fluorescent material can nevertheless pass this mirror since they are reflected from the edges of the glass pipe and exit through the glass pipe. In this manner a good distribution of the outgoing light can be attained. It is also possible to lock in a liquid fluorescent material by means of a glass plug and then provide this with a mirror on that side which faces the exit or the other direction.

The glass pipe itself can also be covered with a mirror on the exit side 10. A narrower light beam will then emerge from the glass pipe, and the conducting part of the cell will then function as a condensator.

A mirror 11 can also be arranged at the other end of the glass pipe 1. This can suitably be a spectrally partial mirror through which light of a certain wave length can pass. This makes it possible to have a light source axially behind the pipe 1, which light source can send light rays of desired wave length through the mirror 11 and excite the dye 9.

The fluorescent cell has been shown as rod-shaped in the present case, but it is understandable that it can also have any other shape which by means of suitable mirrors directs the light out in a desired manner.

As has been said above, the fluorescent cell is fed with light laterally from a lamp as shown in Fig. 1, or axially from a light source at the rear side of the cell 1. Important for the invention is, however, that light is generated in the handheld device and supply means for energy therefor is provided. The supply means suitably consists of an electric cable with a cathode and earth coaxially arranged. Water conduits can also be combined with this cable. Power aggregates which provide electric current of suitable voltage and size and cooling aggregates and the pump for the cooling medium can then be arranged in a suitable place remote from the handheld irradiation device.

By means of the present invention, an arrangement is provided which creates light of desired wave length in the shape of a coherent, converging or diverging light beam which can be held directly against the skin. This arrangement is energy-saving and the total efficiency for the unit increases by about 60% when compared to earlier known units in which the desired light had to be conducted via light conductors to the hand.

The invention is not limited to the embodiments shown, but it can be varied in different ways within the scope of the claims.

## Claims

1. Arrangement for emission of light or laser light, **characterized in** that it comprises a longitudinal cover adapted to be handheld, which cover houses an exchangeable, preferably rod-shaped, fluorescent cell (1) with or without an optical resonator, and supply means for energy to the cell (1) and output means for emitted light (8) or laser light.

2. Arrangement according to claim 1, **charac****terized in** that the cell (1) consists of one or more dyes in plastics or dissolved in a solvent or the like in a glass pipe, plastics pipe or the like, or as a self sustaining element.

3. Arrangement according to claim 2, **charac****terized in** that the plastics consists of polymethylmethacrylate or hydroxypropylacrylate and the solvent of polyethylenglycol, ethylenglycol, ethanol, methanol etc,. and the dye is one which is emitting in the ultra violet, visible or infra red spectrum, for example cumarines or rhodamines.

4. Arrangement according to claim 1, **charac****terized in** that the cell (1) is mounted in the device by means of a rubber ring (7) or the like on the glass pipe (1).

5. Arrangement according to claim 1, **charac****terized in** that the cell (1) is provided with a mirror in at least one end.

6. Arrangement according to claim 5, **characterized in** that at the other end the cell is provided with an annular mirror.

7. Arrangement according to claim 1, **charac****terized in** that the dye cell at the rear end is provided with a spectrally partial mirror (11).

8. Arrangement according to claim 7, **charac****terized in** that the cell (1) is supplied with light energy axially from the rear end.

9. Arrangement according to claim 1, **charac****terized in** that the cell (1) is supplied with light energy laterally from a lamp (2).

10. Arrangement according to claim 1, **charac****terized in** that at its forward end the dye cell (1) is without a mirror while a glass plug in the pipe, which entraps the fluorescent material, is provided with a mirror.

11. Arrangement according to claim 1, **charac****terized in** that the supply means for energy comprises a device for supplying electric energy having high voltage or electric energy having a low voltage or light.
